# EUROPEAN PATENT SPECIFICATION

(11) **EP 2 588 495 B1**
(45) Date of publication and mention of the grant of the patent: **01.04.2015**
(21) Application number: 11728634.4
(22) Date of filing: 30.06.2011
(51) Int. Cl.: C07K 14/47, G01N 33/68, G01N 33/564, C07K 16/18

(54) **Histone citrullinated peptides and uses thereof**
Histoncitrullinierte Peptide und deren Verwendungen
Peptides citrullinés d'histone et leurs utilisations

(30) Priority: 02.07.2010 EP 10168270
(43) Date of publication of application: 08.05.2013
(73) Proprietor: Toscana Biomarkers S.r.l., 53100 Siena (IT)
(72) Inventor: PRATESI, Federico, I-53100 Siena (IT); ALCARO, Maria Claudia, I-53100 Siena (IT); CHELLI, Mario, I-53100 Siena (IT); LOLLI, Francesco, I-53100 Siena (IT); PAOLINI, Ilaria, I-53100 Siena (IT); PAPINI, Anna Maria, I-53100 Siena (IT); ROVERO, Paolo, I-53100 Siena (IT); MIGLIORINI, Paola, I-53100 Siena (IT)
(74) Representative: Capasso, Olga
(86) International application number: PCT/EP2011/061006
(87) International publication number: WO 2012/001103

(56) References cited:
- WO-A2-2004/078098
- WO-A2-2009/147201
- WO-A2-2010/117694
- OSBORNE TANESHA C ET AL: "Protein arginine methyltransferase 1: Positively charged residues in substrate peptides distal to the site of methylation are important for substrate binding and catalysis", BIOCHEMISTRY, vol. 46, no. 46, November 2007 (2007-11), pages 13370-13381, XP002611757, ISSN: 0006-2960 cited in the application
- WYSOCKA JOANNA ET AL: "Histone arginine methylation and its dynamic regulation", FRONTIERS IN BIOSCIENCE, vol. 11, January 2006 (2006-01), pages 344-355, XP002611758, ISSN: 1093-9946 cited in the application
- HAGIWARA TERUKI ET AL: "Deimination of histone H2A and H4 at arginine 3 in HL-60 granulocytes", BIOCHEMISTRY, vol. 44, no. 15, April 2005 (2005-04), pages 5827-5834, XP002611759, ISSN: 0006-2960 cited in the application
- TAM J P: "SYNTHETIC PEPTIDE VACCINE DESIGN SYNTHESIS AND PROPERTIES OF A HIGH-DENSITY MULTIPLE ANTIGENIC PEPTIDE SYSTEM", PROCEEDINGS OF THE NATIONAL ACADEMY OF SCIENCES OF THE UNITED STATES OF AMERICA, vol. 85, no. 15, 1988, pages 5409-5413, XP002611760, ISSN: 0027-8424

### SEQUENCE LISTING

<110> Toscana Biomarkers srl PRATESI, Federico ALCARO, Maria Claudia CHELLI, Mario LOLLI, Francesco PAOLINI, Ilaria PAPINI, Anna Maria ROVERO, Paolo MIGLIORINI, Paola
<120> HISTONE CITRULLINATED PEPTIDES AND USES THEREOF
<130> PCT 112287
<150> EP10168270.6
   <151> 2010-07-02
<160> 3
<170> PatentIn version 3.5
<210> 1
   <211> 50
   <212> PRT
   <213> Artificial Sequence
<220>
   <223> synthetic peptide
<220>
   <221> MISC_FEATURE
   <222> (3)..(3)
   <223> arginine or citrulline residue
<220>
   <221> MISC_FEATURE
   <222> (17)..(17)
   <223> arginine or citrulline residue
<220>
   <221> MISC_FEATURE
   <222> (19)..(19)
   <223> arginine or citrulline residue
<220>
   <221> MISC_FEATURE
   <222> (23)..(23)
   <223> arginine or citrulline residue
<220>
   <221> MISC_FEATURE
   <222> (35)..(35)
   <223> arginine or citrulline residue
<220>
   <221> MISC_FEATURE
   <222> (36)..(36)
   <223> arginine or citrulline residue
<220>
   <221> MISC_FEATURE
   <222> (39)..(39)
   <223> arginine or citrulline residue
<220>
   <221> MISC_FEATURE
   <222> (40)..(40)
   <223> arginine or citrulline residue
<220>
   <221> MISC_FEATURE
   <222> (45)..(45)
   <223> arginine or citrulline residue
<400> 1
   Ser Gly Xaa Gly Lys Gly Gly Lys Gly Leu Gly Lys Gly Gly Ala Lys
   1               5                   10                  15
   Xaa His Xaa Lys Val Leu Xaa Asp Asn Ile Gln Gly Ile Thr Lys Pro
               20                  25                  30
   Ala Ile Xaa Xaa Leu Ala Xaa Xaa Gly Gly Val Lys Xaa Ile Ser Gly
           35                  40                  45
   Leu Ile
       50
<210> 2
   <211> 16
   <212> PRT
   <213> Artificial Sequence
<220>
   <223> synthetic peptide
<400> 2
   Glu Glu Glu Asp Glu Asp Met Gly Phe Gly Leu Phe Asp Lys Lys Ala
   1               5                   10                  15
<210> 3
   <211> 23
   <212> PRT
   <213> Artificial Sequence
<220>
   <223> synthetic peptide
<400> 3
   Arg Lys Val Leu Cys Ile Thr Asp Asn Ile Gln Gly Lys Arg Gln Gly
   1               5                   10                  15
   Cys Ile Thr Thr Leu Tyr Gly
               20

## Description

### Field of invention

The present invention refers to citrullinated synthetic peptides derived from the histone H4 protein and their use in the diagnosis of autoimmune diseases, particularly Rheumatoid Arthritis (RA).

### State of the art

Rheumatoid arthritis (RA) is an inflammatory joint disease characterized by an autoimmune mechanism mediated by both T- and B-cells. Different autoantibodies can be detected in the biological fluids of RA patients. Some of these autoantibodies can be used for RA diagnosis and follow up.

The best known RA biomarker is the rheumatoid factor (RF), a class of IgG or IgM antibodies directed against the Fc-region of the IgG isotype of immunoglobulins. However, RF is not a specific RA marker as it can be found also in other pathological conditions and in elderly healthy individuals. The discovery of the involvement of citrullinated (cit) epitopes in RA using anti-perinuclear factor and anti-keratin antibodies boosted the identification of novel highly specific RA biomarkers. Different citrullinated peptides and proteins have been used to set up specific and sensitive diagnostic assays allowing to recognize RA autoantibodies, thus generally named as anti-citrullinated peptide (CP) antibodies (ACPA) [US20020143143, US20070087380, EP1946109, US20070148704, WO2009007846, WO2009000077, WO2007017556, EP1456662, WO1999028344, WO2008132264]. Among all the proteins associated with RA, the following citrullinated sequences have been used in diagnostic assays: fibrin [C. Masson-Bessière et al. J. Immunol. 2001, 166, 4177], collagen II [H. Burkhardt et al. Eur. J. Immunol. 2005, 35, 1643], vimentin [E.R. Vossenaar et al. Arthritis Rheum. 2004, 50, 3485], filaggrin [US20090028885, US7445903, US6890720], and viral proteins [C. Anzilotti et al. J. Rheumatol. 2006, 33, 647; WO2004087747].

The sensitivity of the tests was increased by the use of filaggrin-derived synthetic cyclic citrullinated peptides (CCPs) [G.A. Schellekens et al. J. Clin. Invest. 1998, 101, 273] [WO1998022503] characterized by a structure that optimally exposes the citrullines for antibody binding. Presently, three generations of CCP tests have been developed: CCP1 (sensitivity 68%, specificity 98%) [G.A. Schellekens et al. Arthritis Rheum. 2000, 43, 155], CCP2 (sensitivity range 39-94%, specificity range 81-100%) [J. Avouac et al. Ann. Rheum. Dis. 2006, 65, 845], and CCP3 (sensitivity range 51-83%, specificity range 93-98%) [L. Lutteri et al. Clin. Chim. Acta 2007, 386, 76]. These RA diagnostic ELISAs are commercially available (Euro-Diagnostica, Arnhem, The Netherlands; Axis-Shield, Dundee, Scotland; INOVA, San Diego, USA).

Recently, the use of citrullinated sequences derived from proteins EBNA-1 (VCP1) and EBNA-2 (VCP2) of Epstein-Barr virus allowed the set up of diagnostic assays characterized by specificities and sensitivities comparable to the ones reported by CCP2 and CCP3 tests [F. Pratesi et al. Arthritis Rheum. 2006, 54, 733; C. Anzilotti et al. J. Rheumatol. 2006, 33, 647; WO2004087747; EP091767764]. Interestingly, the comparison of the performances of the tests based on CCP2, CCP3, VCP1, and VCP2 suggested that different citrullinated sequences can detect different subgroups of anti-CP antibodies in the same set of patients. These results confirmed the important role of the amino acid sequence surrounding citrulline residues in the formation of anti-CP epitopes, in contrast with the hypothesized exclusive role of the XG or XnonG unit (X = citrulline) [EP1693673].

Another class of proteins associated with other autoimmune diseases but not yet directly related to RA diagnosis is represented by histone proteins. Histones are constituents of the nucleosomes and are classified into six classes: H1, H2A, H2B, H3, H4, and H5. Sequences derived from H1 and H2B were used to detect autoantibodies in systemic lupus erythematosus, RA and systemic sclerosis [WO03044054]. Even if it is known that these proteins are natively deiminated and generally modified [K. Arita et al. PNAS 2006, 103, 5291; A.J.W. Zendman et al. Anal. Biochem. 2007, 369, 232], no histone citrullinated sequences have been, up to now, associated with RA. However, microarrays based on the use of modified histones (i.e. phosphorylated, methylated, acetylated, and ubiquitinated), but not citrullinated, were described for the detection of antibodies [WO07132177]. Osborne et al. [Biochemistry, 2007, 46(46), 13370-13381] describe the chemical synthesis of a histone H4 fragment (1-21) having a citrulline residue in positions 17 and 19 for enzymatic studies.

Wysocka et al. [Frontiers in Bioscience, 2006, 11, 344-355] disclose histone H4 being citrullinated in vivo only at position 3 and having citrulline residues in positions 3, 17 and 19, obtainable by in vitro deamination.

Hagiwara et al. [Biochemistry, 2005, 44(15), 5827-5834] disclose the histone H4 fragment (1-23) and (1-5) having Cit in position 3.

WO 20091147201 discloses binding agents, such as antibodies that specifically bind to citrullinated peptides, including enzymatically citrullinated histone H4. These binding agents are used in preventing or treating an autoimmune disease, in particular RA.

WO2010/117694 discloses a sequence that is a combination of histone H4 fragments comprising a citrulline residue at positions 23 and 95.

WO 2004/078098 discloses a peptide not related to histone proteins and mainly consisting of citrulline residues.

### Summary of the invention

The present invention relates to citrullinated synthetic peptides derived from the core histone protein H4 and their use for the diagnosis of autoimmune diseases, in particular, RA. The investigation of the role of histone deimination in RA led to the definition of a series of citrullinated sequences derived from H4. Such sequences are able to recognize autoantibodies specific for RA. The citrullinated peptides according to the invention derive from the amino acid sequence of the human H4, in particular from the sequence comprised between amino acid (aa) 1 to aa 50 (Swiss-Prot P62805 aa 2 to aa 51) of said sequence.

It is therefore an object of the invention a peptide characterized by
- being antigenically effective and able to specifically bind rheumatoid arthritis-specific antibodies and
- consisting, from the amino to the carboxylic terminal, of the amino acid sequence: SGX₁GKGGKGLGKGGAKX₂HX₃K(aa 1 to aa 20 of SEQ ID No. 1), G AKX₂HX₃KVLX₄DNIQGITKPAI(aa 14 to aa 34 of SEQ ID No. 1) or K PAIX₅X₆LAX₇X₈GGVKX₉ISGLI(aa 31 to aa 50 of SEQ ID No. 1) wherein the amino acids X₁-X₉ are selected independently from an arginine residue or a citrulline residue and at least two of X₁-X₉ are a citrulline residue, or a functional fragment thereof being at least 7 amino acid long and comprising at least two citrulline residues said functional fragment being antigenically effective and able to specifically bind rheumatoid arthritis-specific antibodies.

The peptides as described in the invention and possessing at least two citrulline residues were demonstrated to be very suitable for the specific diagnosis of RA. Shorter peptide sequences comprising at least seven a.a. residues and derived from H4(1-50) (Formula I) are of particular interest.

Preferably, the peptide is a functional fragment of SEQ ID No. 1 and consisting of the sequence selected from the group of, G A K Cit H Cit K V L Cit D N I Q G I T K P A I (aa 14 to aa 34 of SEQ ID No. 1 wherein X₂-X₄ are citrulline (Cit) residues,), K P A I Cit Cit L A Cit Cit G G V K Cit I S G L I (aa 31 to aa 50 of SEQ ID No. 1 wherein X₅-X₉ are citrulline (Cit) residues) or a functional fragment thereof being at least 7 amino acid long and comprising at least two citrulline residues said functional fragment being antigenically effective and able to specifically bind rheumatoid arthritis-specific antibodies

Preferably, the peptide as indicated above is of linear form or in the form of multimeric branched peptide or of other conjugated complex.

The multimeric branched peptide preferably consists of:
- a MAP nucleus structure;
- a linear peptide having an amino acid sequence of formula III, IV, and/or V linked through a chemical bond to each of amino terminal residues of the MAP nucleus structure, wherein the linear peptides are equal or different each others. According to the invention a MAP nucleus structure is: 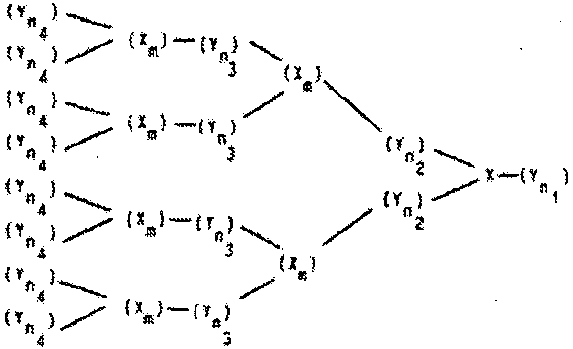
wherein X is an amino acid having at least two amino functional residues, Y is an amino acid selected from the group of alanine and/or glycine and/or a molecule used as spacer, m is 0 or 1, n₁ n₂ n₃ n₄ are integer numbers comprised between 0 and 10, and wherein bonds are carbamido bonds.

A MAP or conjugate comprising a plurality of copies of a citrullinated linear synthetic peptide or of an antigenically effective fragment derived therefrom as described above, bound to an immunologically inert amino-acid core, therefore also falls within the scope of the present invention.

Still preferably, the peptide comprises four identical copies of the peptide as described above.

In a preferred embodiment the peptide is selected from the group of:
(S G Cit G K G G K G L G K G G A K Cit H Cit K)₄ K₂ K beta (Formula VI): 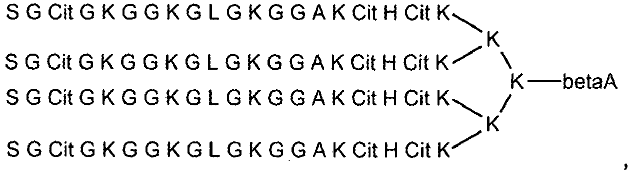
(G A K Cit H Cit K V L Cit D N I Q G I T K P A I)₄ K₂ K beta (Formula VII), 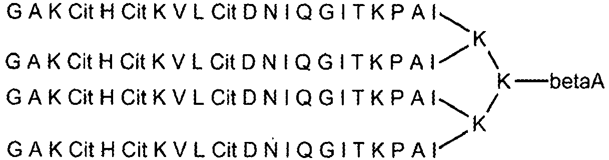 or
(K P A I Cit Cit L A Cit Cit G G V K Cit I S G L I)₄ K₂ K beta (Formula VIII), 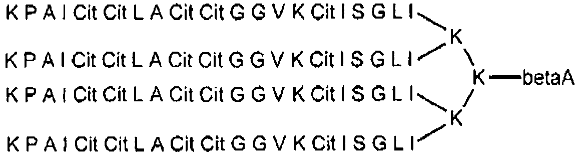 wherein Cit is a citrulline residue and betaA is a beta-alanine residue.

It is a further object of the invention a method for the diagnosis of rheumatoid arthritis in a subject comprising the step of detecting antibodies specific for the autoimmune disease in a biological sample by
- reacting under proper conditions said biological sample with at least one peptide of the invention to produce a complex;
- detecting the complex.

Higher sensitivity and specificity of the method is obtained when the peptides are used in the form of MAPs or of other conjugated complex, in particular in the form of tetravalent MAPs.

It was found that a method based on the use of a MAP of citrullinated peptides of formula VI, VII, or VIII gives the highest sensitivity and specificity in a test for detecting specific RA antibodies. Thus, preferably the biological sample is reacted with at least one peptide of the invention of formula III, IV, V, VI, VII, or VIII. In another preferred embodiment a method is based on the combined use of peptides of formula III, IV, and V or on the combined use of MAP of formula VII and MAP of formula VIII for detecting specific RA antibodies.

Preferably the biological sample is reacted with the peptide comprising the sequence (G A K Cit H Cit K V L Cit D N I (G I T K P A I)₄ K₂ K betaA, 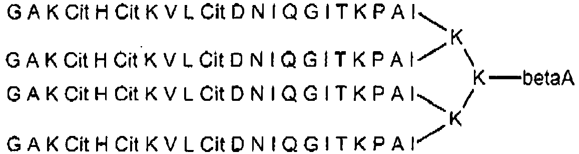 and the peptide comprising the sequence
(K P A I Cit Cit L A Cit Cit G G V K Cit I S G L I)₄ K₂ K betaA, 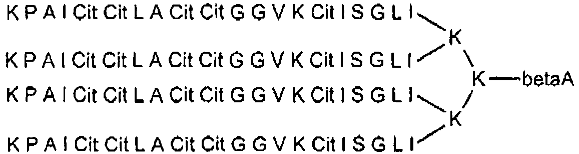
wherein Cit is a citrulline residue and betaA is a beta-alanine residue.

Still preferably the method is an immunological assay.

It is another object of the invention a kit for the diagnostic of rheumatoid arthritis comprising at least one peptide of any one of claim 1 to 6, or a functional fragment thereof being at least 7 amino acid long and comprising at least two citrulline residues said functional fragment being antigenically effective and able to specifically bind rheumatoid arthritis-specific antibodies, said kit detecting antibodies specific for rheumatoid arthritis.

Preferably the kit comprises the peptide comprising the sequence (G A K Cit H Cit K V L Cit D N I Q G I T K P A I)₄ K₂ K betaA, 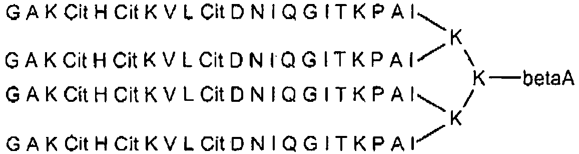 and the peptide comprising the sequence

(K P A I Cit Cit L A Cit Cit G G V K Cit E S G L I)₄ K₂ K betaA, 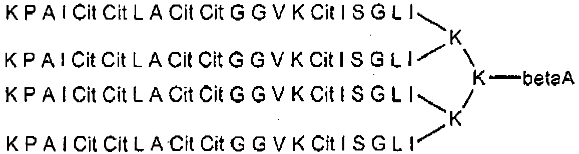 wherein Cit is a citrulline residue and betaA is a beta-alanine residue.

In particular the kit allows the detection of anti-CP antibodies in biological fluids. The kit comprises one of the citrullinated peptides as defined above or a functional fragment thereof, and at least one further reagent. Preferably, the further reagent is an anti-human immunoglobulin conjugated to an enzyme capable of reacting with a chromogenic substrate. The kit may also comprise instructions for use.

It is another object of the invention an antibody directed against the peptide as describe above for use in idagnostic assays for rheumatoid arthritis.

For diagnostic applications, the peptides as described in the invention can be used to detect specific antibodies present in biological fluids of RA patients. In another preferred embodiment, the peptides can be used to detect circulating and tissue- infiltrating auto-reactive B- and T-cells.

In another embodiment of the present invention, the peptides as above disclosed, bound to appropriate resins, can be used for antibody removal.

An antibody present in the biological fluids of autoimmune patients will bind to the peptides of the invention. As indicated above, the citrullinated peptides of the invention, both in linear form and in MAP form, are particularly suitable to be used as antigens in an assay to detect the presence and/or to measure the levels of autoimmune disease-specific antibodies, such as anti-CP antibodies, in a sample of a biological fluid.

In order to perform the assay, the peptides can be adsorbed or covalently linked or modified with a carrier to bind it to a solid support (e.g. chips, microspheres, gold, polystyrene, reactor vessels or wells, micro-titre plate). In a first step of the method, the sample of the biological fluid to be analyzed is placed in contact and incubated with the peptide of the invention that may be linked to the solid support. Autoimmune disease-specific antibody, such as anti-CP antibodies, that are possibly present in the sample are thus specifically bound to the peptide of the invention, producing an antigen/antibody complex. The anti-CP antibodies to be detected in the immunoassay are IgG, IgA, or IgM immunoglobulins. The evaluation of the presence and the quantity of the antigen/antibody complex can be performed with a spectroscopic, a piezoelectric, or an electrochemical biosensor.

The above described method may be an immunological assay in which an indicator antibody, like an anti-human immunoglobulin, is conjugated to an enzyme and is added to measure the antibody titer by a spectroscopic transducer.

It was found that a method based on the use of a MAP of citrullinated peptides of formula VI, VII, or VIII gives the highest sensitivity and specificity in a test for detecting specific RA antibodies. Thus, preferably the biological sample is reacted with at least one peptide of the invention of formula III, IV, V, VI, VII, or VIII. In another preferred embodiment a method is based on the combined use of peptides of formula III, IV, and V or on the combined use of MAP of formula VII and MAP of formula VIII for detecting specific RA antibodies.

In the present invention antigenically effective means that the peptide, or a functional fragment thereof is able to specifically bind autoimmune disease-specific antibodies. In particular, the peptide or the functional fragment thereof is able to specifically bind rheumatoid arthritis-specific antibodies.

### Detailed description of the invention

The invention will be now illustrated by means of non limiting examples referring to the following figures.

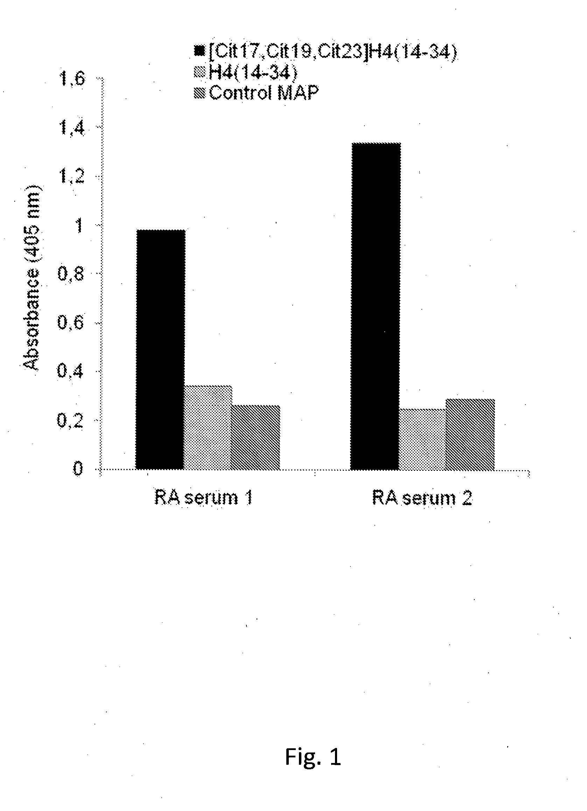

Figure 1. Anti-CP antibodies purified from two different representative sera of RA patients using peptide [Cit¹⁷,Cit¹⁹,Cit²³]H4(14-34) (i.e aa 14 to aa 34 of SEQ ID No. 1 wherein X₂-X₄ are citrulline (Cit) residues, formula IV), the non citrullinated sequence H4(14-34), and an unrelated control MAP of sequence (EEEDEDMGFGLFD)₄-K₂-K-betaA (SEQ ID No. 2). Anti-citrullinated peptide antibodies (ACPA) purification was achieved using the citrullinated peptide [Cit¹⁷,Cit¹⁹,Cit²³]H4(14-34) (formula IV). By contrast, the non citrullinated sequence H4(14-34) and the control MAP did not detect any IgG antibody in the two RA sera.

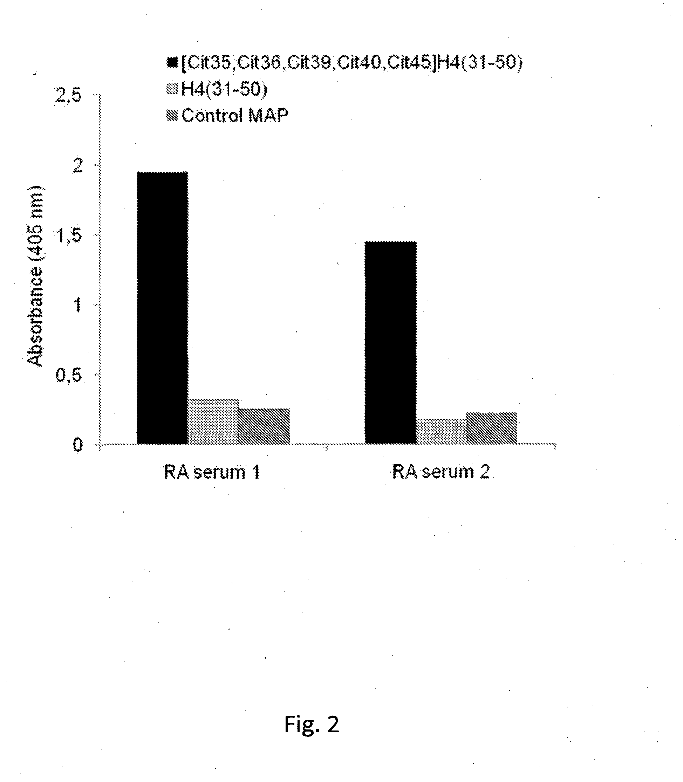

Figure 2. Anti-CP antibodies purified from two different representative sera of RA patients using peptide [Cit³⁵,Cit³⁶,Cit³⁹,Cit⁴⁰,Cit⁴⁵]H4(31-50) (i.e, aa 31 to aa 50 of SEQ ID No. 1 wherein X₅-X₉ are citrulline (Cit) residues, formula V), the non citrullinated sequence H4(31-50), and an unrelated control MAP of sequence (EEEDEDMGFGLFD)₄-K₂-K-betaA (SEQ ID No. 2). ACPA purification was achieved using the citrullinated peptide [Cit³⁵,Cit³⁶,Cit³⁹,Cit⁴⁰,Cit⁴⁵]H4(31-50) (formula V). By contrast, the non citrullinated sequence H4(31-50) and the control MAP did not detect any IgG antibody in the two RA sera.
Figure 3. Mean of the antibody titer for the RA and the NHS groups to differently citrullinated sequences of H4(14-34).
Figure 4. Mean of the antibody titer for the RA and the NHS groups to differently citrullinated sequences of H4(31-50).
Figure 5. Mean of the antibody titer for the RA and the NHS groups to citrullinated overlapping peptides comprised in the H4(1-50).

### EXAMPLES

### Example 1. Peptide Synthesis

Peptides were synthesized using a Wang resin preloaded with the C-terminal amino acid of the sequence or with the MAP core and following the Fmoc/tBu solid-phase peptide strategy [R.B. Merrifield J. Am. Chem. Soc. 1963, 85, 2149; E. Atherton *et al.* Oxford: IRL Press 1989; J.P. Tam Proc. Natl. Acad. Sci. USA 1988, 85, 5409]. Fmoc deprotections were carried out in 20 min with 20% piperidine in DMF. Coupling reactions were performed by treating the resin for 45 min with a 0.5 M solution of the Fmoc-protected amino acids and HOBt in DMF (2.5 equiv), a 0.5 M solution of TBTU in DMF (2.5 equiv), and 4 M NMM in DMF (5 equiv). Peptide cleavage from the resin and deprotection of the amino acid side chains were carried out in 3 h with TFA/thioanisole/ethanedithiol/phenol/H₂O (82.5:5:2.5:5:5). The crude products were precipitated with cold Et₂O, centrifuged, and lyophilized. The pure peptides were obtained by HPLC in a purity >95% and characterized by mass spectrometry (ESI-Orbitrap and/or MALDI-TOF).

### Example 2. ELISA for the determination of anti-citrullinated peptide antibodies

The MAP of the citrullinated peptide antigens according to the invention was diluted to a concentration of 10 µg/ml in phosphate buffered saline (PBS) and loaded into the wells of a polystyrene micro-titration plate (50 µl/well). The plate was left overnight at +4°C to permit interaction between peptide and plastics; however, it may be incubated at 37°C for 1-2 hours with the same result. Upon completion of the coating period, the wells containing the antigen, plus an equal number of wells which were used as controls, were treated for 1 hour at room temperature (RT) with 3% bovine serum albumin (BSA) in PBS. The patients' serum samples (diluted 1:200 in a buffer constituted by 1% BSA, 0.05% Tween X-100 in PBS) were then loaded onto the plate (50 µl/well) and left to incubate for 3 hours at RT. After the incubation period, one washing was performed with 1% PBS Tween X-100 and two washings were performed with PBS (150 µl/well). An anti human-IgG, IgM or IgA antibody conjugated to the enzyme alkaline phosphatase in 1% PBS BSA, 0.05% Tween X-100, was used to show that the antigen/antibody reaction had taken place. The antibody (50 µl/well) was then incubated for 3 hours at RT with agitation. Upon completion of the incubation, after three washings as described above, the alkaline phosphatase substrate (p-nitrophenyl phosphate) was added to the wells and, in the presence of the enzyme, it produced a yellow product measurable by spectrophotometric techniques at a wavelength of 405 nm; its quantity was proportional to the titre of antibodies bound. The results of the test were expressed as the percentage of positivity, calculated by dividing the absorbance of each serum sample by the absorbance of a positive serum sample the value of which was set arbitrarily at 100.

Serum samples of 97 patients suffering from RA and of 34 normal healthy subjects (NHS) were tested by this method using the peptide of formula III, IV, or V.

A result that was greater than 97.5^{th} percentile of the normal control sera group is defined as positive, data are reported in Table 1.

**Table 1. Number of positive sera over total sera tested for peptides of formula III, IV, and V.**

| Formula | RA (% RA positive sera) | NHS |
|---|---|---|
| III | 20/97 (21%) | 2/34 |
| IV | 10/97 (10%) | 2/34 |
| V | 12/97 (12%) | 2/34 |

Serum samples of 101 patients suffering from RA and of 48 NHS were tested by this method using the MAPs of formula VI, VII, or VIII.

Moreover, 91 serum samples of disease controls other than RA (systemic sclerosis, mixed cryoglobulinemia, systemic lupus erythematosus, ankylosing spondylitis, and psoriatic arthritis) were tested by this method using MAPs of formula VI, VII or VIII, which showed the most interesting reactivities.

A result that was greater than 97.5^{th} percentile of the normal control sera group (NHS) is defined as positive, data are reported in Table 2.

**Table 2. Number of positive sera over total sera tested for MAP peptides of formula VI, VII, and VIII.**

| Formula | RA positive sera | NHS | Ssc | MC | SLE | AS | PA |
|---|---|---|---|---|---|---|---|
| VI | 28/101 | 2/48 | 3/7 | 1/12 | 6/48 | 5/12 | 1/12 |
| VII | 65/101 | 2/48 | 1/7 | 0/12 | 3/48 | 0/12 | 0/12 |
| VIII | 61/101 | 1/48 | 0/7 | 0/12 | 1/48 | 1/12 | 0/12 |

| | | | | | | | |
|---|---|---|---|---|---|---|---|
| *wherein: RA = rheumatoid arthritis, NHS = normal healthy subjects, Ssc = systemic sclerosis, MC = mixed cryoglobulinemia, SLE = systemic lupus erythematosus, AS = ankylosing spondylitis, PA = psoriatic arthritis. | | | | | | | |

The overall sensitivity and specificity of the three assays are shown in the following Table 3.

**Table 3. Sensitivity and specificity of the assays based on the peptide of formula VI, VII, VIII.**

| Formula | Sensitivity | Specificity** |
|---|---|---|
| VI | 28% | 85% |
| VII | 65% | 96% |
| VIII | 60% | 98% |

| | | |
|---|---|---|
| **Determined on the NHS and the disease control populations indicated in Table 2 | | |

An epitope mapping of the full protein H4 was performed and the data showed that the N-terminal part, H4(1-50), comprising peptide of formula I differently citrullinated corresponded to the antigenic portion of the molecule. In particular, the citrullinated C-terminal portion of H4(1-50) is highly reactive and data reported in Tables 1-3 demonstrate that the sequences of formula III-VIII can be used to recognize RA sera.

It is noteworthy that until now, histones and their citrullination have never been associated with RA, whereas reactivity towards antibodies present in SLE patients' sera is well known [WO03044054]. Data reported in Table 3 demonstrate a very good sensitivity and specificity of sequences of formula VII and VIII for RA. Very interestingly and surprisingly, neither the MAP of formula VII nor the MAP of formula VIII showed a significant reactivity in detecting specific antibodies in SLE patients sera. Therefore, the authors of the present invention demonstrate the unexpected correlation between citrullinated peptide sequences comprising the H4(1-50) region and the specific recognition of autoantibodies in RA patients sera. Thus, such peptides are biomarkers of the disease.

In addition, the sequences object of the present invention don't overlap any one of the proteins or peptides already used to detect anti-CP antibodies (e.g. fibrin, collagen II, vimentin, filaggrin, viral proteins, and synthetic CCPs). This demonstrates the innovative role of the peptide sequences derived from H4(1-50) to recognize anti-CP antibodies with 63-67% of sensitivity and 96-97% of specificity in the case of MAPs of formula VII and VIII.

### Example 3. Diagnostic properties of an ELISA based on the contemporary use of the MAP of formula VII and of the MAP of formula VIII

To achieve a highly sensitive immunoassay, an equimolar mixture of MAP of formula VII and MAP of formula VIII was allowed to adsorb to 96-wells microtitre plates for 4 h at r.t. The test was then performed as described in example 2. Analysing a second independent population of 147 RA patients tested for antibodies towards MAP peptides of formula VII and VIII, the authors found that:
- 87/147 (59%) RA patients recognize the peptide of formula VII;
- 89/147 (61%) RA patients recognize the peptide of formula VIII.

Analysing more in details this RA patients population positive either to peptide of formula VII or to peptide of formula VIII the authors found that:
- 71/147 (48%) react with both peptides of formula VII and VIII;
- 16/147 (11%) sera are positive only to the peptide of formula VII;
- 18/147 (12%) sera are positive only to the peptide of formula VIII

Thus, the combined use of citrullinated peptide MAP of formula VII and citrullinated MAP of formula VIII improves the diagnostic performances of the test in respect of the single peptide based assays. The combined use leads to a RA high sensitive diagnostic assay (105/147 - 71,5% RA positive sera) as the two antibody populations identified in RA sera are overlapped but different.

### Example 4. Purification of anti-citrullinated peptides antibodies and uses thereof

Anti-CP antibodies can be purified from serum of RA patients by means of affinity chromatography procedures.

A citrullinated peptide or MAP according to the present invention was conjugated to CNBr-activated sepharose according to standard procedures known by one skilled in the art. Total immunoglobulins from sera containing anti-CP antibodies were precipitated with 50% saturated ammonium sulfate; the precipitates were dissolved in phosphate buffer (pH 7.4) and dialyzed overnight against PBS. Enriched immunoglobulin preparations were applied to the column, and the flow through was collected for subsequent analysis. The column was extensively washed with 20 mM Na₂HPO₄, 150 mM NaCl (pH 7.2), and the antibodies bound to the column were eluted by 0.1 M glycine buffer (pH 2.8) (0.5 ml/fraction), immediately neutralized with 50 µl Tris 1 M (pH 8.0), and dialyzed overnight against PBS. The anti-CP antibody content in the eluates and flowthrough was tested by ELISA (Fig. 1 and 2).

Figure 1 shows that ACPA purification was achieved using the citrullinated peptide [Cit¹⁷,Cit¹⁹,Cit²³]H4(14-34) (formula IV). By contrast, the non citrullinated sequence H4(14-34) and the control MAP did not detect any IgG antibody in the two RA sera. Figure 2 shows that ACPA purification was also achieved using the citrullinated peptide [Cit³⁵,Cit³⁶,Cit³⁹,Cit⁴⁰,Cit⁴⁵]H4(31-50) (formula V). By contrast, the non citrullinated sequence H4(31-50) and the control MAP did not detect any IgG antibody in the two RA sera.

Such purified antibodies can be used as controls in solid-phase assays using citrullinated antigens.

### Example 5. Role of the number and position of citrullines.

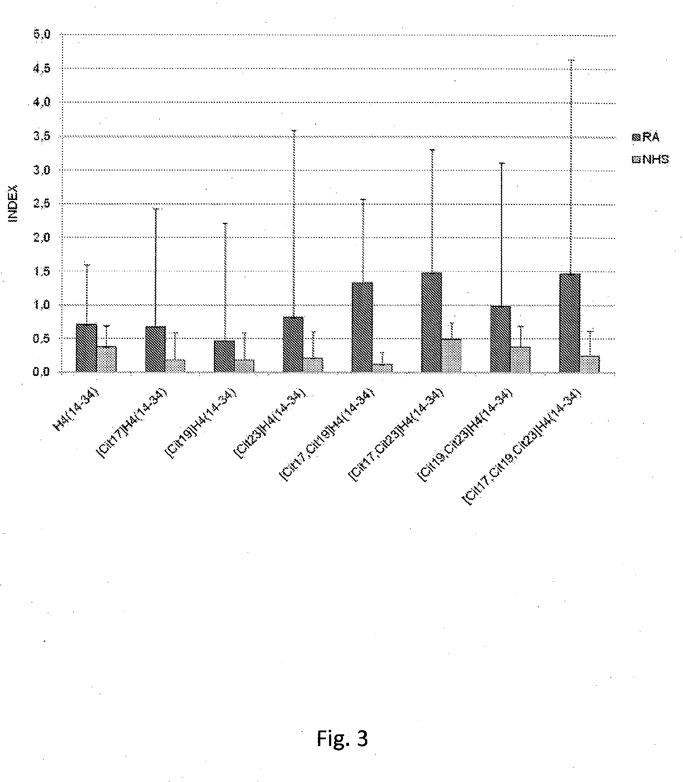

The role of the number and of the position of citrullines inside H4(1-50) sequence in the detection of specific antibodies in RA sera was investigated by means of differently citrullinated peptides (Figures 3-4).

**Table 4: Citrullinated peptides**

| Sequence name | Sequence |
|---|---|
| H4(14-34) | GAKRHRKVLRDNIQGITKPAI (aa 14 to aa 34 of SEQ ID No. 1 wherein X₂-X₄ are arginine residues) |
| [Cit¹⁷]H4(14-34) | GAKCitHRKVLRDNIQGITKPAI (aa 14 to aa 34 of SEQ ID No. 1 wherein X₂ is Cit and X₃-X₄ are arginine residues) |
| [Cit¹⁹]H4(14-34) | GAKRHCitKVLRDNIQGITKPAI (aa 14 to aa 34 of SEQ ID No. 1 wherein X₃ is Cit and X₂ and X₄ are arginine residues) |
| [Cit²³]H4(14-34) | GAKRHRKVLCitDNIQGITKPAI (aa 14 to aa 34 of SEQ ID No. 1 wherein X₄ is Cit and X₂ and X₃ are arginine residues) |
| [Cit¹⁷,Cit¹⁹]H4(14-34) | GAKCitHCitKVLRDNIQGITKPAI (aa 14 to aa 34 of SEQ ID No. 1 wherein X₂ and X₃ are Cit and X₄ is arginine) |
| [Cit¹⁷,Cit²³]H4(14-34) | GAKCitHRKVLCitDNIQGITKPAI (aa 14 to aa 34 of SEQ ID No. 1 wherein X₂ and X₄ are Cit and X₃ is arginine |
| [Cit¹⁹,Cit²³]H4(14-34) | GAKRHCitKVLCitDNIQGITKPAI (aa 14 to aa 34 of SEQ ID No. 1 wherein X₃ and X₄ are Cit and X₂ is arginine |
| [Cit¹⁷,Cit¹⁹,Cit²³]H4(14-34) | GAKCitHCitKVLCitDNIQGITKPAI, formula IV |
| H4(31-50) | KPAIRRLARRGGVKRISGLI (aa 31 to aa 50 of SEQ ID No. 1 wherein X₅-X₉ are arginine residues) |
| [Cit³⁵]H4(31-50) | KPAICitRLARRGGVKRISGLI (aa 31 to aa 50 of SEQ ID No. 1 wherein X₅ is Cit and X₆-X₉ are arginine residues) |
| [Cit³⁶]H4(31-50) | KPAIRCitLARRGGVKRISGLI (aa 31 to aa 50 of SEQ ID No. 1 wherein X₆ is Cit and X₅ and X₇-X₉ are arginine residues) |
| [Cit³⁹]H4(31-50) | KPAIRRLACitRGGVKRISGLI (aa 31 to aa 50 of SEQ ID No. 1 wherein X₇ is Cit and X₅-X₆ and X₈-X₉ are arginine residues) |
| [Cit⁴⁰]H4(31-50) | KPAIRRLARCitGGVKRISGLI (aa 31 to aa 50 of SEQ ID No. 1 wherein X₈ is Cit and X₅-X₇ and X₉ are arginine residues) |
| [Cit⁴⁵]H4(31-50) | KPAIRRLARRGGVKCitISGLI (aa 31 to aa 50 of SEQ ID No. 1 wherein X₉ is Cit and X₅-X₈ are arginine residues) |
| [Cit³⁵,Cit⁴⁰]H4(31-50) | KPAICitRLARCitGGVKRISGLI (aa 31 to aa 50 of SEQ ID No. 1 wherein X₅ and X₈ are Cit and X₆-X₇ and X₉ are arginine residues) |
| [Cit³⁶,Cit⁴⁰]H4(31-50) | KPAIRCitLARCitGGVKRISGLI (aa 31 to aa 50 of SEQ ID No. 1 wherein X₆ and X₈ are Cit and X₅, X₇ and X₉ are arginine residues) |
| [Cit³⁹,Cit⁴⁰]H4(31-50) | KPAIRRLACitCitGGVKRISGLI (aa 31 to aa 50 of SEQ ID No. 1 wherein X₇ and X₈ are Cit and X₅-X₆ and X₉ are arginine residues) |
| (Cit⁴⁰,Cit⁴⁵]H4(31-50) | KPAIRRLARCitGGVKCitlSGLI (aa 31 to aa 50 of SEQ ID No. 1 wherein X₈ and X₉ are Cit and X₅-X₇ are arginine residues) |
| [Cit³⁵,Cit³⁶,Cit⁴⁰]H4(31-50) | KPAICitCitLARCitGGVKRISGLI (aa 31 to aa 50 of SEQ ID No. 1 wherein X₅, X₆ and X₈ are Cit and X₇ and X₉ are arginine residues) |
| [Cit³⁵,Cit³⁹,Cit⁴⁰]H4(31-50) | KPAICitRLACitCitGGVKRISGLI (aa 31 to aa 50 of SEQ ID No. 1 wherein X₅, X₇ and X₈ are Cit and X₆ and X₉ are arginine residues) |
| [Cit³⁵,Cit⁴⁰,Cit⁴⁵]H4(31-50) | KPAICitRLARCitGGVKCitISGLI (aa 31 to aa 50 of SEQ ID No. 1 wherein X₅, X₈ and X₉ are Cit and X₆ and X₇ are arginine residues) |
| [Cit³⁶,Cit³⁹,Cit⁴⁰]H4(31-50) | KPAIRCitLACitCitGGVKRISGLI (aa 31 to aa 50 of SEQ ID No. 1 wherein X₆, X₇ and X₈ are Cit and X₅ and X₉ are arginine residues) |
| [Cit³⁶,Cit⁴⁰,Cit⁴⁵]H4(31-50) | KPAIRCitLARCitGGVKCitISGLI (aa 31 to aa 50 of SEQ ID No. 1 wherein X₆, X₈ and X₉ are Cit and X₅ and X₇ are arginine residues) |
| [Cit³⁵,Cit³⁶,Cit³⁹,Cit⁴⁰]H4(31-50) | KPAICitCitLACitCitGGVKRISGLI (aa 31 to aa 50 of SEQ ID No. 1 wherein X₅-X₈ are Cit and X₉ is arginine) |
| [Cit³⁵,Cit³⁶,Cit⁴⁰,Cit⁴⁵]H4(31-50) | KPAICitCitLARCitGGVKCitISGLI (aa 31 to aa 50 of SEQ ID No. 1 wherein X₅-X₆ and X₈-X₉ are cit and X₇ is arginine) |
| [Cit³⁵,Cit³⁹,Cit⁴⁰,Cit⁴⁵]H4(31-50) | KPAICitRLACitCitGGVKCitISGLI (aa 31 to aa 50 of SEQ ID No. 1 wherein X₅, X₇ and X₈-X₉ are Cit and X₆ is arginine) |
| [Cit³⁶,Cit³⁹,Cit⁴⁰,Cit⁴⁵]H4(31-50) | KPAIRCitLACitCitGGVKCitISGLI (aa 31 to aa 50 of SEQ ID No. 1 wherein X₆-X₉ are Cit and X₅ is arginine) |
| [Cit³⁵,Cit³⁶,Cit³⁹,Cit⁴⁰,Cit⁴⁵]H4(31-50) | KPAICitCitLACitCitGGVKCitISGLI, formula V |
| [Cit³,Cit¹⁷,Cit¹⁹]H4(1-20) | SGCitGKGGKGLGKGGAKCitHCitK, formula III |
| [Cit²³]H4(19-28) | RKVLCitDNIQG (aa 19 to aa 28 of SEQ ID No. 1 wherein X₄ is Cit and X₃ is arginine) |
| [Cit¹⁹,Cit²³,Cit³⁵,Cit³⁶]H4(19-38) | CitKVLCitDNIQGITKPAICitCitLA (aa 19 to aa 38 of SEQ ID No. 1 wherein X₃-X₆ are Cit) |
| [Cit²³,Cit³⁵,C³⁶,Cit³⁹,Cit⁴⁰]H4(22-41) | LCitDNIQGITKPAICitCitLACitCitG (aa 22 to aa 41 of SEQ ID No. 1 wherein X₄-X₈ are Cit) |
| [Cit²³,Cit⁹⁵]H4(19-28)-(91-99) | RKVLCitDNIQGKRQGCitTLYG SEQ ID No. 3 |

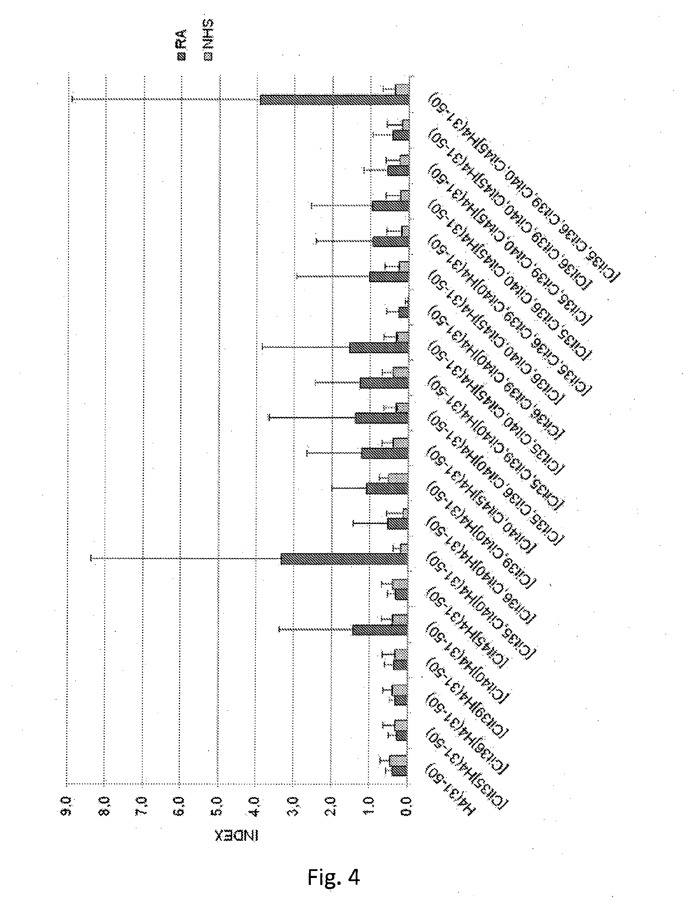

Serum samples of 14 RA patients and 23 NHS were tested by ELISA using the method described in example 2. Results were analyzed determining the cutoff value as the OD mean of the NHS sera plus two standard deviations and then expressed as index (OD sample / OD cutoff). Results are positive if the index is above 1,0. The mean of the antibody titer for the RA and the NHS groups to the differently citrullinated peptides H4(14-34) and H4(31-50) is reported in Figures 3 and 4, respectively.

For the sequence H4(14-34) it was found that at least two citrullines are necessary to discriminate between RA and NHS patients (Figure 3). As expected, the non citrullinated H4(14-34) did not show any reactivity. In addition, the introduction of a single citrulline residue in the H4(14-34) showed that one deiminated arginine is not enough to allow the detection of ACPA. This result suggests an active role of the amino acids flanking the citrulline in antibody recognition.

By contrast, the introduction of two citrulline residues allows the discrimination between RA and NHS patients.

In particular, the peptide [Cit¹⁷,Cit²³]H4(14-34) is the most active di-citrullinated peptide. Therefore, for the sequence H4(14-34), the presence of two citrullines is the minimal requirement for the recognition of ACPA in RA sera. It should be noted that the best discrimination between RA and NHS sera is achieved when all the three arginine present in the H4(14-34) sequence are deiminated.

Data reported in Figure 4 for H4(31-50) confirmed that the absence of citrullines did not allow to detect any ACPA and to discriminate between RA and NHS. The introduction of one citrulline showed a reactivity only for [Cit⁴⁰]H4(31-50), suggesting a possible role of deimination of position 40 in ACPA recognition. Among the di-citrullinated peptides, [Cit³⁵,Cit⁴⁰]H4(31-50) is the most reactive sequence. This finding strongly demonstrates the addictive effect of two citrullines. H4(31-50) derivatives containing three or four citrullines are able to discriminate between RA and NHS sera but are less preferred because not as sensitive as [Cit³⁵,Cit⁴⁰]H4(31-50) and [Cit³⁵,Cit³⁶,Cit³⁹,Cit⁴⁰,Cit⁴⁵]H4(31-50) (formula V).

### Example 6. Analysis of the antigenic determinants.

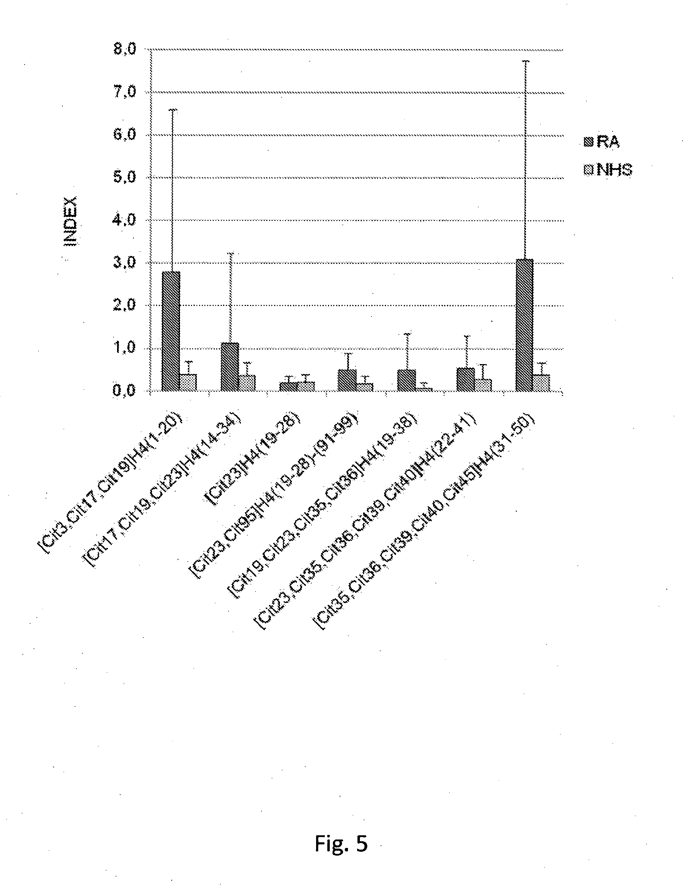

The presence of the antigenic determinants inside H4(1-50) sequence was investigated using overlapping peptides selected in order to contain the maximum number of citrullines per sequence (Table 5 and Figure 5).

**Table 5. Overlapping peptides used to define the antigenic determinants in H4(1-50).**

| | |
|---|---|
| [Cit³,Cit¹⁷,Cit¹⁹]H4 (1-20) | SGXGKGGKGLGKGGAKXHXK |
| [Cit¹⁷,Cit¹⁹,Cit²³]H4(14-34) | GAKXHXKVLXDNIQGITKPAI |
| [Cit²³]H4(19-28) | RKVLXDNIQG |
| [Cit¹⁹,Cit²³,Cit³⁵,Cit³⁶]H4 (19-38) | XKVLXDNIQGITKPAIXXLA |
| [Cit²³,Cit³⁵,Cit³⁶,Cit³⁹,Cit⁴⁰]H4(22-41) | LXDNIQGITKPAIXXLAXXG |
| [Cit³⁵,Cit³⁶,Cit³⁹,Cit⁴⁰, Cit⁴⁵]H4(31-50) | KPAIXXLAXXGGVKXISGLI |

| | |
|---|---|
| X = Cit | |

The study was performed on the linear peptides: [Cit³,Cit¹⁷,Cit¹⁹]H4(1-20) (aa 1 to aa 20 of SEQ ID No. 1 wherein X₁-X₃ are citrulline (Cit) residues, formula III), [Cit¹⁷,Cit¹⁹,Cit²³]H4(14-34) (aa 14 to aa 34 of SEQ ID No. 1 wherein X₂-X₄ are citrulline (Cit) residues, formula IV), [Cit²³]H4(19-28) (aa 19 to aa 28 of SEQ ID No. 1 wherein X₄ is a citrulline residue), [Cit¹⁹,Cit²³,Cit³⁵,Cit³⁶]H4(19-38) (aa 19 to aa 38 of SEQ ID No. 1 wherein X₃-X₆ are a citrulline residue), [Cit²³,Cit³⁵,Cit³⁶,Cit³⁹,Cit⁴⁰]H4(22-41) (aa 22 to aa 41 of SEQ ID No. 1 wherein X₄-X₈ are a citrulline residue), [Cit³⁵,Cit³⁶,Cit³⁹,Cit⁴⁰,Cit⁴⁵]H4(31-50) (aa 31 to aa 50 of SEQ ID No. 1 wherein X₅-X₉ are citrulline (Cit) residues, formula V).

Serum samples of 18 RA patients and 23 NHS were tested by ELISA using the method described in example 2. Results were analyzed determining the cutoff value as the OD mean of the NHS sera plus two standard deviations and then expressed as index (OD sample / OD cutoff). Results are positive if the index is above 1,0. The mean of the antibody titer for the RA and the NHS groups to peptides is reported in Figure 5.

Discrimination between RA and NHS sera was obtained with peptides of formulae III-V, but not with [Cit¹⁹,Cit²³,Cit³⁵,Cit³⁶]H4(19-38) or [Cit²³,Cit³⁵,Cit³⁶,Cit³⁹,Cit⁴⁰]H4(22-41). Interestingly, the sequences [Cit¹⁹,Cit²³,Cit³⁵,Cit³⁶]H4(19-38) and [Cit²³,Cit³⁵,Cit³⁶,Cit³⁹,Cit⁴⁰]H4(22-41) are less reactive although they contain more citrullines than [Cit³,Cit¹⁷,Cit¹⁹]H4(1-20) and [Cit¹⁷,Cit¹⁹,Cit³]H4(14-34). This result suggests the importance of the amino acids flanking the citrulline residues. The peptide [Cit³⁵,Cit³⁶,Cit³⁹,Cit⁴⁰,Cit⁴⁵]H4(31-50) (formula V) showed the best reactivity in this series.

In this experiment, the peptide [Cit²³,Cit⁹⁵]H4(19-28)-(91-99), Arg¹⁹-Lys-Val-Leu-Cit-Asp-Asn-Ile-Gln-Gly²⁸-Lys⁹¹-Arg-Gln-Gly-Cit-Thr-Leu-Tyr-Gly⁹⁹ (SEQ ID No. 4), was synthesized and tested on the same set of sera to compare its reactivity with the molecules object of the present invention and comprised in the H4(1-50) sequence. No significant discrimination between the RA and NHS sera was observed using [Cit²³,Cit⁹⁵]H4(19-28)-(91-99) or using the peptide [Cit²³]H4(19-28). This data suggest that the antigenic determinants in the histone H4 protein are located in the 1-50 region.

Considering also the data reported in Table 3, the presence of two different antigenically effective domains can be hypothesized to be located inside the H4(1-50) in the 17-23 and 35-45 regions.

## Claims

1. A peptide **characterized by**
- being antigenically effective and able to specifically bind rheumatoid arthritis-specific antibodies and
- consisting, from the amino to the carboxylic terminal, of the amino acid sequence:
S G X₁ G K G G K G L G K G G A K X₂ H X₃ K (aa 1 to aa 20 of SEQ ID No. 1), G A K X₂ H X₃ K V L X4 D N I Q G I T K P A I (aa 14 to aa 34 of SEQ ID No. 1) or K P A I X₅ X₆ LA X₇ X₈ G G V K X₉ I S G L I (aa 31 to aa 50 of SEQ ID No. 1) wherein the amino acids X₁-X₉ are selected independently from an arginine residue or a citrulline residue and at least two of X₁-X₉ are a citrulline residue, or a functional fragment thereof being at least 7 amino acid long and comprising at least two citrulline residues said functional fragment being antigenically effective and able to specifically bind rheumatoid arthritis-specific antibodies.

2. The peptide of claim 1 consisting of the sequence selected from the group of, G A K Cit H Crt K V L Cit D N I Q G I T K P A I (aa 14 to aa 34 of SEQ ID No. 1 wherein X₂-X₄ are citrulline (Cit) residues), K P A I Cit Cit L A Cit Cit G G V K Cit I S G L I (aa 31 to aa 50 of SEQ ID No. 1 wherein X₅-X₉ are citrulline (Cit) residues) or a functional fragment thereof being at least 7 amino acid long and comprising at least two citrulline residues said functional fragment being antigenically effective and able to specifically bind rheumatoid arthritis-specific antibodies.

3. The peptide according to any one of preceding claims being of linear form.

4. The peptide according to any one of claim 1 to 2, being in the form of multimeric branched peptide.

5. The peptide of claim 4 comprising four identical copies of the peptide according to claim 1 to 2.

6. The peptide of claim 5 being selected from the group of:
(S G Cit G K G G K G L G K G G A K Cit H Cit K)₄ K₂ K betaA. 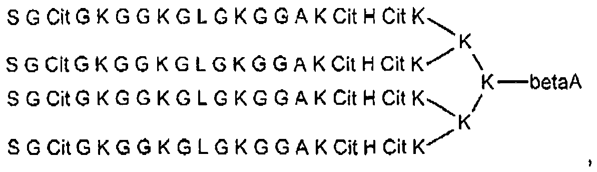
(G A K Cit H Cit K V L Cit D N I Q G I T K P A I)₄ K₂ K betaA, 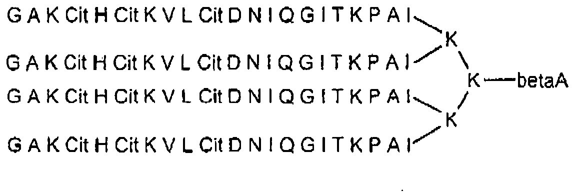 or
(K P A I Cit Cit L A Cit Cit G G V K Cit I S G L I)₄ K₂ K betaA, 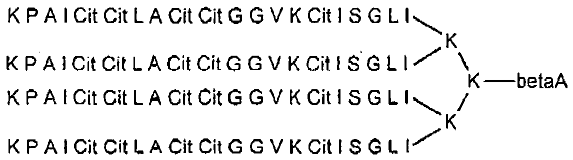
wherein Cit is a citrulline residue and betaA is a beta-alanine residue.

7. A method for the diagnosis of rheumatoid arthritis in a subject comprising the step of detecting antibodies specific for rheumatoid arthritis in a biological sample by
- reacting under proper conditions said biological sample with at least one peptide of any one of claim 1 to 6 to produce a complex;
- detecting the complex.

8. The method of claim 7 wherein the biological sample is reacted with the peptide comprising the sequence (G A K Cit H Cit K V L Cit D N I Q G I T K P A I)₄ K₂ K betaA, 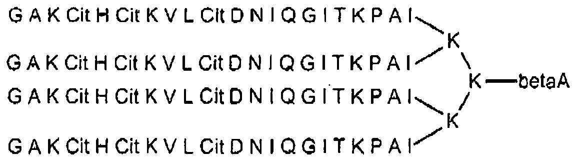 and the peptide comprising the sequence
(K P A I Cit Cit L A Cit Cit G G V K Cit I S G L l)₄ K₂ K betaA, 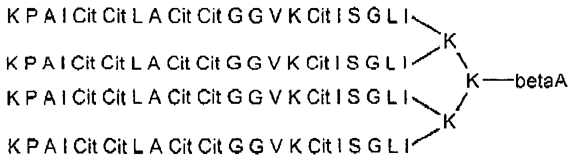
wherein Cit is a citrulline residue and betaA is a beta-alanine residue.

9. The method of any one of claims 7 or 8 being an immunological assay.

10. A kit for the diagnostic of rheumatoid arthritis comprising at least one peptide of any one of claim 1 to 6, or a functional fragment thereof being at least 7 amino acid long and comprising at least two citrulline residues said functional fragment being antigenically effective and able to specifically bind rheumatoid arthritis-specific antibodies, said kit detecting antibodies specific for rheumatoid arthritis.

11. The kit of claim 10 comprising the peptide comprising the sequence (G A K Cit H Cit K V L Cit D N I Q G I T K P A I)₄ K₂ K betaA, 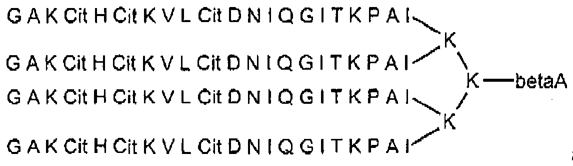 and the peptide comprising the sequence
(K P A I Cit Cit LA Cit Cit G G V K Cit I S G L I)₄ K₂ K betaA, 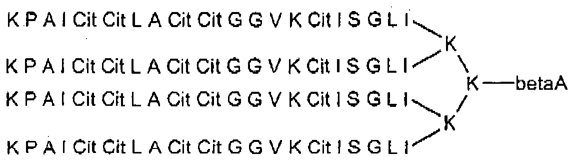
wherein Cit is a citrulline residue and betaA is a beta-alanine residue.

12. An antibody directed against the peptide of any one of claims 1 to 6 for use in diagnostic assays for rheumatoid arthritis.

## Patentansprüche

1. Peptid, **dadurch gekennzeichnet, dass** es
- antigen wirksam und zur spezifischen Bindung von Antikörpern, die spezifisch für rheumatoide Arthritis sind, in der Lage ist und
- vom Amino- bis zum Carboxyterminus aus der folgenden Aminosäuresequenz besteht:
S G X₁ G K G G K G L G K G G A K X₂ H X₃ K (AS 1 bis AS 20 von SEQ ID Nr. 1), G A K X₂ H X₃ K V L X₄ D N I Q G I T K P A I (AS 14 bis AS 34 von SEQ ID Nr. 1) oder K P A I X₅ X₆ L A X₇ X₈ G G V K X₉ I S G L I (AS 31 bis AS 50 von SEQ ID Nr. 1), wobei die Aminosäuren X₁-X₉ unabhängig aus einem Argininrest oder einem Citrullinrest ausgewählt sind und mindestens zwei von X₁-X₉ ein Citrullinrest sind, oder ein funktionelles Fragment davon, das mindestens 7 Aminosäuren lang ist und mindestens zwei Citrullinreste umfasst, wobei das funktionelle Fragment antigen wirksam und zur spezifischen Bindung von Antikörpern, die spezifisch für rheumatoide Arthritis sind, in der Lage ist.

2. Peptid nach Anspruch 1, das aus der Sequenz besteht, die aus der Gruppe G A K Cit H Cit K V L Cit D N I Q G I T K P A I (AS 14 bis AS 34 von SEQ ID Nr. 1, wobei X₂-X₄ Citrullinreste (Cit) sind), K P A I Cit Cit L A Cit Cit G G V K Cit I S G L I (AS 31 bis AS 50 von SEQ ID Nr. 1, wobei X₅-X₉ Citrullinreste (Cit) sind) ausgewählt ist, oder ein funktionelles Fragment davon, das mindestens 7 Aminosäuren lang ist und mindestens zwei Citrullinreste umfasst, wobei das funktionelle Fragment antigen wirksam und zur spezifischen Bindung von Antikörpern, die spezifisch für rheumatoide Arthritis sind, in der Lage ist.

3. Peptid nach einem der vorhergehenden Ansprüche, das von linearer Form ist.

4. Peptid nach einem der Ansprüche 1 bis 2, das in der Form eines multimeren verzweigten Peptids vorliegt.

5. Peptid nach Anspruch 4, umfassend vier identische Kopien des Peptids nach Anspruch 1 bis 2.

6. Peptid nach Anspruch 5, das ausgewählt ist aus der Gruppe:
(S G Cit G K G G K G L G K G G A K Cit H Cit K)4 K₂ K betaA, 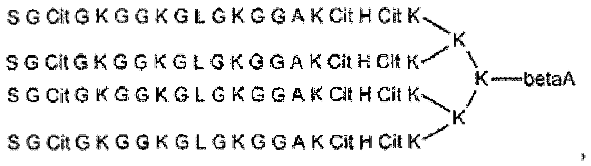
(G A K Cit H Cit K V L Cit D N I Q G I T K P A I)₄ K₂ K betaA, 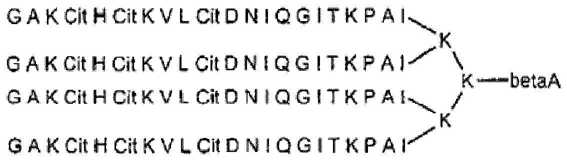 und
(K P A I Cit Cit L A Cit Cit G G V K Cit I S G L I)₄ K₂ K betaA, 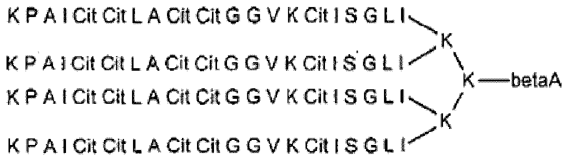 wobei Cit ein Citrullinrest ist und betaA ein Beta-Alanin-Rest ist.

7. Verfahren für die Diagnose von rheumatoider Arthritis bei einem Individuum, umfassend den Schritt des Detektierens von Antikörpern, die für rheumatoide Arthritis spezifisch sind, in einer biologischen Probe durch
- In Reaktion bringen der biologischen Probe mit mindestens einem Peptid nach einem der Ansprüche 1 bis 6 unter entsprechenden Bedingungen, um einen Komplex herzustellen;
- Detektieren des Komplexes.

8. Verfahren nach Anspruch 7, wobei die biologische Probe mit dem Peptid umfassend die Sequenz (G A K Cit H Cit K C L Cit D N I Q G I T K P A I)₄ K₂ K betaA, 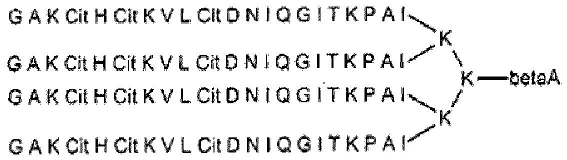 und dem Peptid umfassend die Sequenz (K P A I Cit Cit L A Cit Cit G G V K Cit I S G L I)₄ K₂ K betaA. 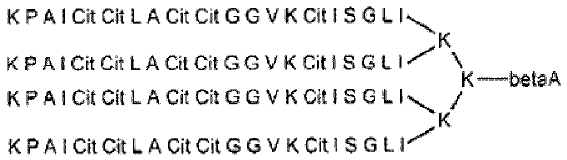 in Reaktion gebracht wird, wobei Cit ein Citrullinrest ist und betaA ein Beta-Alanin-Rest ist.

9. Verfahren nach einem der Ansprüche 7 oder 8, bei dem es sich um einen immunologischen Assay handelt.

10. Kit für die Diagnose von rheumatoider Arthritis, umfassend mindestens ein Peptid nach einem der Ansprüche 1 bis 6 oder ein funktionelles Fragment davon, das mindestens 7 Aminosäuren lang ist und mindestens zwei Citrullinreste umfasst, wobei das funktionelle Fragment antigen wirksam und zur spezifischen Bindung von Antikörpern, die spezifisch für rheumatoide Arthritis sind, in der Lage ist, wobei das Kit Antikörper detektiert, die spezifisch für rheumatoide Arthritis sind.

11. Kit nach Anspruch 10, umfassend das Peptid umfassend die Sequenz (G A K Cit H Cit K C L Cit D N I Q G I T K P A I)₄ K₂ K betaA, 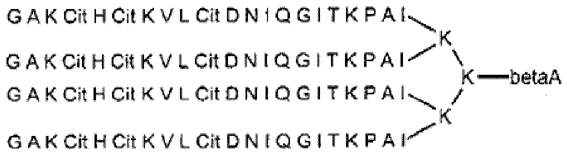 und das Peptid umfassend die Sequenz (K P A I Cit Cit L A Cit Cit G G V K Cit I S G L I)₄ K₂ K betaA, 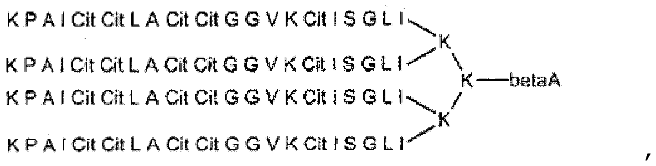 wobei Cit ein Citrullinrest ist und betaA ein Beta-Alanin-Rest ist.

12. Antikörper, der gegen das Peptid nach einem der Ansprüche 1 bis 6 gerichtet ist, zur Verwendung in diagnostischen Assays auf rheumatoide Arthritis.

## Revendications

1. Peptide **caractérisé en ce que**
- il est antigéniquement efficace et capable de spécifiquement lier les anticorps spécifiques de la polyarthrite rhumatoïde et
- il est constitué, de l'extrémité amino à l'extrémité carboxylique, de la séquence d'acides aminés :
S G X₁ G K G G K G L G K G G A K X₂ H X₃ K (aa 1 à aa 20 de SEQ IDNO: 1), G A K X₂ H X₃ K V L X₄ D N I Q G I T K P A I (aa 14 à aa 34 de SEQ ID NO: 1) ou K P A I X₅ X₆ L A X₇ X₈ G G V K X₉ I S G L I (aa 31 à aa 50 de SEQ ID NO: 1), dans laquelle les acides aminés X₁ X₉ sont indépendamment sélectionnés parmi un résidu arginine ou un résidu citrulline, et au moins deux de X₁ X₉ sont un résidu citrulline, ou un fragment fonctionnel de celle-ci étant d'une longueur d'au moins 7 acides aminés et comprenant au moins deux résidus citrulline, ledit fragment fonctionnel étant antigéniquement efficace et capable de spécifiquement lier les anticorps spécifiques de la polyarthrite rhumatoïde.

2. Peptide selon la revendication 1, consistant en la séquence sélectionnée dans le groupe de, G A K Cit H Cit K V L Cit D N I Q G I T K P A I (aa 14 à aa 34 de SEQ ID NO: 1 dans laquelle X₂ X₄ sont des résidus citrulline (Cit)), K P A I Cit Cit L A Cit Cit G G V K Cit I S G L I (aa 31 à aa 50 de SEQ ID NO: 1 dans laquelle X₅ X₉ sont des résidus citrulline (Cit)) ou un fragment fonctionnel de celle-ci étant d'une longueur d'au moins 7 acides aminés, et comprenant au moins deux résidus citrulline, ledit fragment fonctionnel étant antigéniquement efficace et capable de spécifiquement lier les anticorps spécifiques de la polyarthrite rhumatoïde.

3. Peptide selon l'une quelconque des revendications précédentes, étant de forme linéaire.

4. Peptide selon l'une quelconque des revendications 1 et 2, étant sous la forme d'un peptide ramifié multimère.

5. Peptide selon la revendication 4, comprenant quatre copies identiques du peptide selon la revendication 1 ou 2.

6. Peptide selon la revendication 5, étant sélectionné dans le groupe constitué de :
(S G Cit G K G G K G L G K G G A K Cit H Cit K)₄ K₂ K bêtaA, 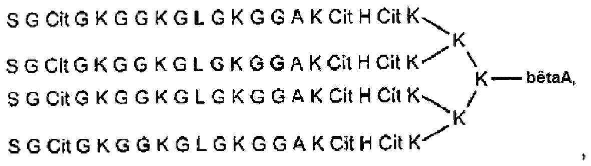
(G A K Cit H Cit K V L Cit D N I Q G I T K P A I)₄ K₂ K bêtaA, 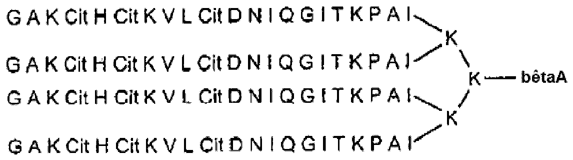 ou
(K P A I Cit Cit L A Cit Cit G G V K Cit I S G L I)₄ K₂ K bêtaA, 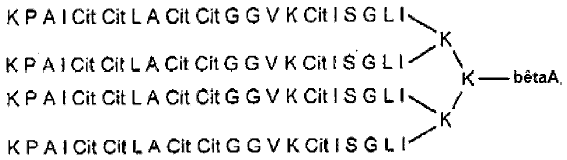 dans lequel Cit est un résidu citrulline et bêtaA est un résidu bêta-alanine.

7. Méthode de diagnostic de la polyarthrite rhumatoïde chez un sujet comprenant l'étape de détection des anticorps spécifiques de la polyarthrite rhumatoïde dans un échantillon biologique par :
- mise en réaction dans des conditions adaptées dudit échantillon biologique avec au moins un peptide selon l'une quelconque des revendications 1 à 6 pour produire un complexe ; et
- détection du complexe.

8. Méthode selon la revendication 7, dans laquelle l'échantillon biologique est mis en réaction avec le peptide comprenant la séquence (G A K Cit H Cit K V L Cit D N I Q G I T K P A I)₄ K₂ K bêtaA, 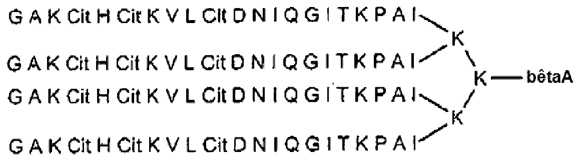 et le peptide comprenant la séquence
(K P A I Cit Cit L A Cit Cit G G V K Cit I S G L I)₄ K₂ K bêtaA, 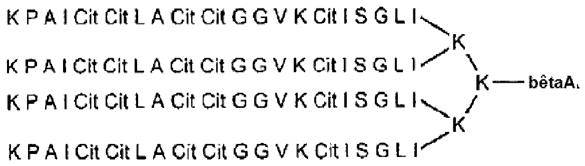 dans lequel Cit est un résidu citrulline et bêtaA est un résidu bêta-alanine.

9. Procédé selon l'une quelconque des revendications 7 ou 8, étant un test immunologique.

10. Kit de diagnostic de la polyarthrite rhumatoïde comprenant au moins un peptide selon l'une quelconque des revendications 1 à 6, ou un fragment fonctionnel de celui-ci étant d'une longueur d'au moins 7 acides aminés, et comprenant au moins deux résidus citrulline, ledit fragment fonctionnel étant antigéniquement efficace et capable de spécifiquement lier les anticorps spécifiques de la polyarthrite rhumatoïde, ledit kit détectant les anticorps spécifiques de la polyarthrite rhumatoïde.

11. Kit selon la revendication 10, comprenant le peptide comprenant la séquence (G A K Cit H Cit K V L Cit D N I Q G I T K P A I)₄ K₂ K bêtaA, 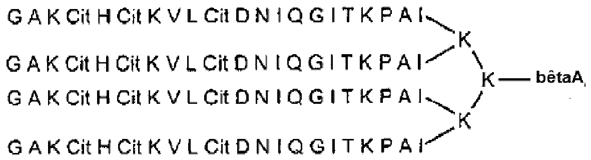 et le peptide comprenant la séquence
(K P A I Cit Cit L A Cit Cit G G V K Cit I S G L I)₄ K₂ K bêtaA, 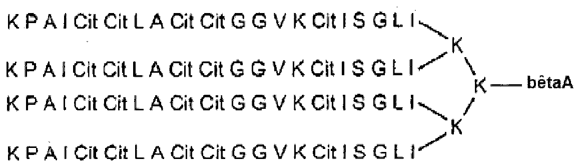 dans lequel Cit est un résidu citrulline et bêtaA est un résidu bêta-alanine.

12. Anticorps dirigé contre le peptide selon l'une quelconque des revendications 1 à 6, pour son utilisation dans des tests diagnostiques de la polyarthrite rhumatoïde.
